# EUROPÄISCHE PATENTSCHRIFT

(11) **EP 1 007 507 B1**
(45) Veröffentlichungstag und Bekanntmachung des Hinweises auf die Patenterteilung: **06.11.2002**
(21) Anmeldenummer: 98941417.2
(22) Anmeldetag: 13.08.1998
(51) Int. Cl.: C07C 253/14, C07C 255/47

(54) **VERFAHREN ZUR HERSTELLUNG VON 2-CYANOINDAN-1-ONEN**
PROCESS FOR PREPARING 2-CYANOINDAN-1-ONES
PROCEDE DE PREPARATION DE 2-CYANOINDANE-1-ONES

(30) Priorität: 25.08.1997 DE 19736921
(43) Veröffentlichungstag der Anmeldung: 14.06.2000
(73) Patentinhaber: Bayer Aktiengesellschaft, 51368 Leverkusen (DE)
(72) Erfinder: KOMOSCHINSKI, Joachim, D-51061 Köln (DE); FIEGE, Helmut, D-51373 Leverkusen (DE); STEFFAN, Guido, D-51519 Odenthal (DE)
(86) Internationale Anmeldenummer: EP9805142
(87) Internationale Veröffentlichungsnummer: WO99010315

(56) Entgegenhaltungen:
- DE-A- 2 640 358
- DE-B- 1 083 250
- FR-A- 1 310 392
- US-A- 2 715 137
- W.S. JOHNSON ET AL: J. AM. CHEM. SOC. , Bd. 67, 1945, Seiten 1745-1754, XP002093927 in der Anmeldung erwähnt
- H. GALONS ET AL: C. R. SEANCES ACAD. SCI., SER. 2, Bd. 292, Nr. 23, 1981, Seiten 1517-1519, XP002093928
- W.S. JOHNSON ET AL: J. AM. CHEM. SOC., Bd. 66, 1944, Seiten 218-221, XP002093929 in der Anmeldung erwähnt
- H. KOBLER ET AL: LIEBIGS ANN. CHEM., Nr. 12, 1978, Seiten 1946-1962, XP002093930

## Beschreibung

Die vorliegende Erfindung betrifft ein verbessertes Verfahren zur Herstellung von 2-Cyanoindan-1-onen aus entsprechenden 2-Halogenindan-1-onen durch Umsetzung mit Cyanid-Salzen.

2-Cyanoindan-1-one sind Vorprodukte für Wirkstoffe, wie sie z.B. in WO 96/20151 und WO 95/29171 beschrieben sind.

Es ist bereits bekannt, daß man 2-Cyanoindan-1-on und 2-Cyano-5-methoxyindan-1-on durch Umsetzung der entsprechenden 2-Bromindan-1-one mit Hilfe von Cyanid-Salzen erhalten kann. So wird in J. Am. Chem. Soc. 66, 220 (1944) und 67, 1751 (1945) die Herstellung dieser Cyanoindan-1-one durch Zusammengeben von 2-Bromindan-1-on bzw. 2-Brom-5-methoxyindan-1-on, Natriumcyanid oder Kaliumcyanid, Ethanol und Wasser, und Kochen dieser Mischung am Rückfluß beschrieben. Hierbei werden bestenfalls Ausbeuten von 52 bzw. 73 % erhalten. Außerdem wird ein 10-facher molarer Überschuß an Cyanid eingesetzt, was erheblichen arbeitshygienischen Aufwand bei der Aufarbeitung bedeutet.

Es wurde nun ein Verfahren zur Herstellung von 2-Cyanoindan-1-onen der Formel 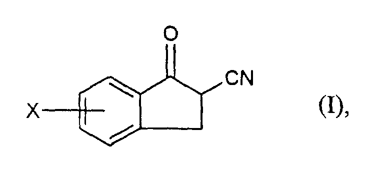 in der
- X: für Wasserstoff, Halogen, Methyl, Trifluormethyl oder Methoxy steht,
aus 2-Halogenindan-1-onen der Formel 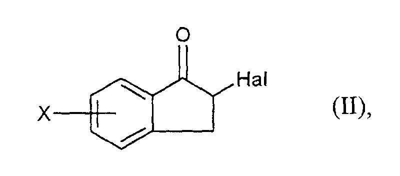 in der
- X: die bei Formel (I) angegebene Bedeutung hat und
- Hal: für Chlor oder Brom steht,
durch Umsetzung mit einem Cyanidsalz gefunden, das dadurch gekennzeichnet ist, daß man das Cyanidsalz gelöst in einem dipolar aprotischen Lösungsmittel oder einem mit Wasser mischbaren Ether vorlegt und dieser Lösung das 2-Halogenindan-1-on der Formel (II) zudosiert.

Halogen bedeutet vorzugsweise Fluor, Chlor oder Brom.

In den Formeln (I) und (II) steht X vorzugsweise für Chlor, insbesondere für Chlor in 5- oder 6-Stellung. Hal in Formel (II) steht vorzugsweise für Chlor.

Als Cyanidsalze kommen z.B. Alkalicyanide und Tetraalkylammoniumcyanide in Frage. Beispiele sind Natrium-, Kalium-, Lithium-, Rubidium-, Tetraethylammonium- und Tetrabutylammoniumcyanid. Das jeweilige Cyanidsalz kann man, bezogen auf 1 Mol der Verbindung der Formel (II), beispielsweise in einer Menge von 1,5 bis 6 Mol einsetzen. Vorzugsweise beträgt diese Menge 1,6 bis 5 Mol, insbesondere 1,8 bis 3 Mol.

Als dipolar aprotische Lösungsmittel kommen z.B. N-Methylpyrrolidon, Dimethylformamid, Dimethylacetamid, Dimethylsulfoxid, Hexamethylphosphorsäuretriamid, Sulfolan und Acetonitril in Frage, als mit Wasser mischbare Ether beispielsweise Tetrahydrofuran und Diglykoldimethylether. Bevorzugt sind Dimethylformamid, Dimethylacetamid, N-Methylpyrrolidon und Dimethylsulfoxid.

Bezogen auf 1 Mol Cyanidsalz kann man z.B. 200 bis 2500 ml dipolar aprotische Lösungsmittel oder mit Wasser mischbare Ether einsetzen. Vorzugsweise liegt diese Menge bei 300 bis 1500 ml.

Die Zudosierung des 2-Halogenindan-1-ons der Formel (II) erfolgt vorzugsweise so, daß man es in gelöster Form nach und nach der vorgelegten Cyanidsalz-Lösung zufügt. Als Lösungsmittel für das 2-Halogenindan-1-on kommen auch dipolar aprotische Lösungsmittel und mit Wasser mischbare Ether in Frage, wie oben ausführlicher beschrieben. Vorzugsweise verwendet man zum Lösen des Cyanidsalzes und zur Bereitung der Lösung des 2-Halogenindan-1-ons das gleiche Lösungsmittel.

Weiterhin ist es bevorzugt, das 2-Halogenindan-1-on als 10 bis 30 gew.-%ige, insbesondere als 15 bis 25 gew.-%ige Lösung einzusetzen.

Die Cyanidsalz-Lösung und/oder die 2-Halogenindan-1-on-Lösung können gegebenenfalls Wasser enthalten. Beispielsweise können, bezogen auf das Lösungsmittel, bis zu 30 Gew.-% Wasser vorhanden sein. Vorzugsweise liegt diese Menge bei 2 bis 10 Gew.-%.

Das erfindungsgemäße Verfahren kann gegebenenfalls unter Zusatz von Säuren zur pH-Kontrolle durchgeführt werden. Bei hohen pH-Werten besteht die Gefahr, daß neben dem Austausch Halogen gegen Cyano eine Abspaltung von Halogenwasserstoff stattfindet. Es ist deshalb vorteilhaft, gegebenenfalls durch Zusatz einer starken Säure den pH-Wert zu kontrollieren und ihn beispielsweise im Bereich 4 bis 11, vorzugsweise 5 bis 10 zu halten. Zu starkes Ansäuern ist zu vermeiden, da sich sonst aus dem Cyanidsalz Blausäure bildet.

Die Säure kann dem Reaktionsgemisch z.B. als solche zugesetzt werden, aber z.B. auch zusammen mit dem 2-Halogenindan-1-on bzw. dessen Lösung.

Als Säuren kommen insbesondere mittelstarke bis starke wäßrige Säuren in Frage wie wäßrige Salzsäure, Bromwasserstoffsäure, Schwefelsäure, Phosphorsäure, Ameisensäure und Essigsäure.

Das erfindungsgemäße Verfahren kann z.B. bei Temperaturen im Bereich 10 bis 70°C durchgeführt werden. Bevorzugt sind Temperaturen im Bereich 20 bis 60°C.

Das nach der Durchführung des erfindungsgemäßen Verfahrens vorliegende Reaktionsgemisch kann man z.B. aufarbeiten, indem man es abkühlt, vorhandene Lösungsmittel bei vermindertem Druck abzieht, den hinterbleibenden Kristallbrei mit Wasser vermischt, schwach ansäuert und den dann vorliegenden Feststoff abfiltriert und mit Wasser wäscht. Gegebenenfalls kann man das so erhaltene 2-Cyanoindan-1-on der Formel (I) weiter reinigen, z.B. durch Umkristallisation aus einem organischen Lösungsmittel.

Mit dem erfindungsgemäßen Verfahren kann man 2-Cyanoindan-1-one der Formel (I) in überraschend hohen Ausbeuten von 95 % und mehr erhalten und mit wesentlich geringeren Cyanidmengen auskommen als bei den Verfahren des Standes der Technik.

### Beispiele

### Beispiel 1

10,4 g Natriumcyanid wurden in einer Mischung aus 100 ml Dimethylformamid und 6 ml Wasser gelöst und vorgelegt. Diese Lösung wurde unter Rühren auf 50°C erwärmt, dann tropfte man 20,4 g 2,5-Dichlorindan-1-on, gelöst in 100 ml Dimethylformamid, innerhalb von 60 Minuten hinzu. Nach vollständiger Zugabe wurde noch 1 Stunde bei 50°C weitergerührt Dann ließ man das Gemisch auf Raumtemperatur abkühlen und zog das Lösungsmittel am Rotationsverdampfer ab. Der so erhaltene Kristallbrei wurde mit 200 ml Wasser vermischt und mit 10 gew.-%iger wäßriger Salzsäure bis zu einem pH-Wert von 4,5 angesäuert Der ausgefallene Feststoff wurde abgesaugt, mit Wasser gewaschen, aus Toluol rekristallisiert und im Vakuumtrockenschrank getrocknet. Auf diese Weise wurden 18,5 g 2-Cyano-5-chlorindan-1-on erhalten (Ausbeute: 97 % d.Th.).

### Beispiel 2

Man arbeitete wie in Beispiel 1 beschrieben, setzte jedoch anstelle von 2,5-Dichlorindan-1-on 2,6-Dichlorindan-1-on ein. 2-Cyano-6-chlorindan-1-on wurde in einer Ausbeute von 95,5 % d. Th. erhalten.

### Beispiel 3 (zum Vergleich)

Zu einer Lösung von 4,2 g 2,5-Dichlorindan-1-on in 140 ml Methanol wurden zunächst 9,6 g Natriumcyanid hinzugerügt und dann soviel Wasser, bis eine homogene Lösung entstand. Diese Mischung wurde 45 Minuten auf dem Wasserbad am Rückfluß gekocht und anschließend auf Raumtemperatur abgekühlt. Nach dem Verdünnen mit weiterem Wasser wurde zweimal mit Diethylether extrahiert. Die zurückbleibende wäßrige Lösung wurde mit kalter Salzsäure angesäuert Das rohe 2-Cyano-5-chlorindan-1-on wurde abgetrennt und mit Chloroform wieder aufgenommen. Diese Lösung wurde nun mit Norit geklärt und anschließend so lange mit kleinen Portionen einer 5 gew.-%igen wäßrigen Natriumhydroxidlösung extrahiert, bis beim Ansäuern einer Probe keine Fällung mehr beobachtet wurde. Durch Ansäuern der vereinigten wäßrigen Phasen erhielt man 2,85 g Produkt, welches nach Rekristallisation aus Toluol 2,7 g 2-Cyano-5-chlorindan-1-on ergab (Ausbeute; 70 % d. Th.).

## Patentansprüche

1. Verfahren zur Herstellung von 2-Cyanoindan-1-onen der Formel 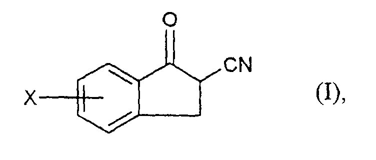 in der
X für Wasserstoff, Halogen, Methyl, Trifluormethyl oder Methoxy steht,
aus 2-Halogenindan-1-onen der Formel 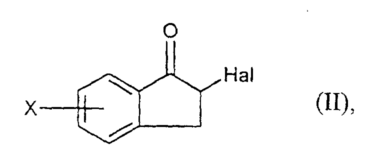 in der
X die bei Formel (I) angegebene Bedeutung hat und
Hal für Chlor oder Brom steht,
durch Umsetzung mit einem Cyanidsalz, **dadurch gekennzeichnet, daß** man das Cyanidsalz gelöst in einem dipolar aprotischen Lösungsmittel oder einem mit Wasser mischbaren Ether vorlegt und dieser Lösung das 2-Halogenindan-1-on der Formel (II) zudosiert.

2. Verfahren nach Anspruch 1, **dadurch gekennzeichnet, daß** in den Formeln X und Hal jeweils für Chlor stehen.

3. Verfahren nach Ansprüchen 1 bis 2, **dadurch gekennzeichnet, daß** bezogen auf 1 Mol einer Verbindung der Formel (II) 1,6 bis 6 Mol Cyanidsalz eingesetzt werden.

4. Verfahren nach Ansprüchen 1 bis 3, **dadurch gekennzeichnet, daß** man als dipolar aprotische Lösungsmittel bzw. mit Wasser mischbare Ether N-Methylpyrrolidon, Dimethylformamid, Dimethylacetamid, Dimethylsulfoxid, Hexamethylphosphorsäuretriamid, Sulfolan, Acetonitril, Tetrahydrofuran oder Diglykoldimethylether einsetzt.

5. Verfahren nach Ansprüchen 1 bis 4, **dadurch gekennzeichnet, daß** man das 2-Halogenindan-1-on der Formel (II) in gelöster Form nach und nach der vorgelegten Cyanidsalz-Lösung zufügt.

6. Verfahren nach Ansprüchen 1 bis 5, **dadurch gekennzeichnet, daß** man das 2-Halogenindan-1-on der Formel (II) als 10 bis 30 gew.-%ige Lösung einsetzt.

7. Verfahren nach Ansprüchen 1 bis 6, **dadurch gekennzeichnet, daß** bezogen auf das Lösungsmittel bis zu 30 Gew.-% Wasser vorhanden sind.

8. Verfahren nach Ansprüchen 1 bis 7, **dadurch gekennzeichnet, daß** man durch Zusatz einer starken Säure den pH-Wert im Bereich 4 bis 11 hält.

9. Verfahren nach Ansprüchen 1 bis 8, **dadurch gekennzeichnet, daß** man es bei 10 bis 70°C durchführt.

10. Verfahren nach Ansprüchen 1 bis 9, **dadurch gekennzeichnet, daß** man das nach der Umsetzung vorliegende Reaktionsgemisch aufarbeitet, indem man es abkühlt, vorhandene Lösungsmittel bei vermindertem Druck abzieht, den hinterbleibenden Kristallbrei mit Wasser vermischt, schwach ansäuert und den Feststoff abfiltriert und mit Wasser wäscht.

## Claims

1. Process for preparing 2-cyanoindan-1-ones of the formula 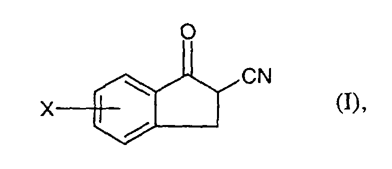 in which
X represents hydrogen, halogen, methyl, trifluoromethyl or methoxy,
from 2-halogenoindan-1-ones of the formula 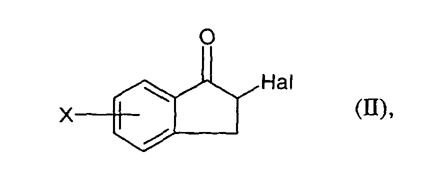 in which
X is as defined under formula (I) and
Hal represents chlorine or bromine,
by reaction with a cyanide salt, **characterized in that** the cyanide salt is initially charged dissolved in a dipolar aprotic solvent or in a water-miscible ether and the 2-halogenoindan-1-one of the formula (II) is metered into this solution.

2. Process according to Claim 1, **characterized in that** in the formulae X and Hal each represent chlorine.

3. Process according to Claims 1 to 2, **characterized in that** from 1.6 to 6 mol of cyanide salt are employed per mole of a compound of the formula (II).

4. Process according to Claims 1 to 3, **characterized in that** the dipolar aprotic solvent or water-miscible ether used is N-methylpyrrolidone, dimethylformamide, dimethylacetamide, dimethyl sulphoxide, hexamethylphosphoric triamide, sulpholane, aceionitrile, tetrahydrofuran or diglycol dimethyl ether.

5. Process according to Claims 1 to 4, **characterized in that** the 2-halogenoindan-1-one of the formula (II) is added in the dissolved form and a little at a time to the cyanide salt solution which has been initially charged.

6. Process according to Claims 1 to 5, **characterized in that** the 2-halogenoindan-1-one of the formula (II) is employed as a 10 to 30% by weight strength solution.

7. Process according to Claims 1 to 6, **characterized in that**, based on the solvent, up to 30% by weight of water are present.

8. Process according to Claims 1 to 7, **characterized in that** the pH is kept in the range from 4 to 11 by addition of a strong acid.

9. Process according to Claims 1 to 8, **characterized in that** it is carried out at from 10 to 70°C.

10. Process according to Claims 1 to 9, **characterized in that** the reaction mixture which is present after the reaction is worked up **in that** it is cooled, any solvents present are stripped off under reduced pressure, the crystal slurry that remains is mixed with water and acidified slightly and the solid is filtered off and washed with water.

## Revendications

1. Procédé pour la préparation de 2-cyanoindan-1-ones de la formule : 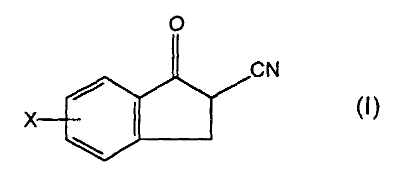 dans laquelle
X représente l'atome d'hydrogène, un atome d'halogène, le radical méthyle, trifluorométhyle ou méthoxy,
à partir de 2-halogénoindan-1-ones de formule : 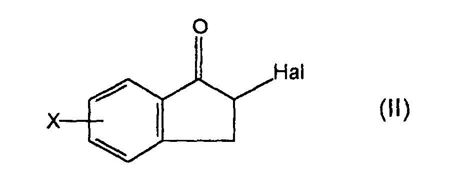 dans laquelle
X a la signification donnée pour la formule (I), et
Hal représente l'atome de chlore ou de brome,
par réaction avec un sel de cyanure, **caractérisé en ce que** l'on dispose le sel de cyanure dissous dans un solvant dipolaire aprotique ou un éther miscible à l'eau et que l'on ajoute à cette solution, en dosant, la 2-halogénoindan-1-one de formule (II).

2. Procédé suivant la revendication 1, **caractérisé en ce que** dans les formules, X et Hal représentent chacun l'atome de chlore.

3. Procédé suivant les revendications 1 et 2, **caractérisé en ce que** sur base de 1 mole d'un composé de formule (II), on met en oeuvre 1,6 à 6 moles de sel de cyanure.

4. Procédé suivant les revendications 1 à 3, **caractérisé en ce que** l'on met en oeuvre comme solvant dipolaire aprotique ou éther miscible à l'eau, la N-méthylpyrrolidone, le diméthylformamide, le diméthylacétamide, le diméthylsulfoxyde, l'hexaméthylphosphotriamide, le sulfolanne, l'acétonitrile, le tétrahydrofuranne et le diglycoldiméthyléther.

5. Procédé suivant les revendications 1 à 4, **caractérisé en ce que** l'on ajoute la 2-halogénoindan-1-one de formule (II) peu à peu, sous forme dissoute, à la solution de sel de cyanure préparée.

6. Procédé suivant les revendications 1 à 5, **caractérisé en ce que** l'on met en oeuvre la 2-halogénoindan-1-one de formule (II) sous forme d'une solution de 10 à 30% en poids.

7. Procédé suivant les revendications 1 à 6, **caractérisé en ce que** sur base du solvant, jusqu'à 30% en poids d'eau sont présents.

8. Procédé suivant les revendications 1 à 7, **caractérisé en ce que** l'on maintient le pH dans l'intervalle allant de 4 à 11 par addition d'un acide fort.

9. Procédé suivant les revendications 1 à 8, **caractérisé en ce qu'**on le réalise dans l'intervalle allant de 10 à 70°C.

10. Procédé suivant les revendications 1 à 9, **caractérisé en ce que** l'on traite le mélange réactionnel présent après la réaction **en ce qu'**on le refroidit, on élimine le solvant présent sous pression réduite, on mélange la bouillie cristalline restante avec de l'eau, on acidifie légèrement, on filtre le solide et on lave avec de l'eau.
